# EUROPEAN PATENT APPLICATION

(11) **EP 1 741 449 A1**
(43) Date of publication of application: **10.01.2007**
(21) Application number: 05727933.3
(22) Date of filing: 31.03.2005
(51) Int. Cl.: A61K 48/00, A61K 35/76, A61K 39/395, A61P 35/00, C07K 14/47, C07K 16/18, C12N 1/15, C12N 1/19, C12N 1/21, C12N 5/10, C12N 15/09, G01N 33/15, G01N 33/50, C12P 21/08

(54) **PREVENTIVE AND/OR REMEDY FOR CANCER**

(30) Priority: 31.03.2004 JP 2004102681
(71) Applicant: Mitsubishi Pharma Corporation, Osaka-shi, Osaka 541-0046 (JP)
(72) Inventor: YOSHIKAWA, Tsutomu, Mitsubishi Pharma Corporation, Tokyo 1038405 (JP); WATANABE, Wakako, Mitsubishi Pharma Corporation, Tokyo 1038405 (JP); HIRAKAWA, Yoko, Mitsubishi Pharma Corporation, Tokyo 1038405 (JP)
(74) Representative: Oser, Andreas
(86) International application number: PCT/JP2005/006241
(87) International publication number: WO 2005/094900

(57) **Abstract**

As the results of intensive studies aiming at providing a gene targeting a cancer cell or a caner tissue, novel nucleotide sequences showing increased expression in a cancer cell or a caner tissue compared with normal tissues are identified. As the results of the subsequent studies, genes showing increased expression in a cancer cell or a caner tissue which are useful as a drug discovery target are found out.

## Description

### Technical Field

The present invention relates to novel nucleotide sequences and use of cancer-related genes. Further, the present invention relates to a method of screening a drug using the cancer-related genes.

### Background Art

In the field of diagnosis and treatment of cancer, for obtaining a sufficient therapeutic effect and reducing side effects of a remedy, a targeting therapy that targets a certain cancer cell has been studied. In particular, there is a method including identifying an antigen specifically expressed on the surface of malignant cells, obtaining a monoclonal antibody against the antigen, and using the monoclonal antibody as a remedy for cancer (Non-patent Document 1 or 2).

In recent years, it has become possible to specify a gene that shows an increased expression in cancer cells or cancer tissues in comparison with normal tissues. Therefore, for identifying a gene to be targeted for diagnosis or treatment of cancer, search for a gene showing expression in cancer tissues higher than in normal tissues has been conducted using a microarray, which is a comprehensive measuring device for gene expression as typified by a biotip array. For example, 69 kinds of genes highly expressed in pancreatic cancer were tried to be identified using BioExpress (Gene Logic Co., Ltd.) in Patent Document 1, and genes whose expression varies in pulmonary adenocarcinoma were tried to be identified in Patent Document 2.

However, any of those existing reports only makes a comparison between cancer tissues and corresponding normal tissues (e.g., normal pancreatic tissue against pancreatic cancer tissue), and not less than several tens to several hundreds genes have been identified as candidate genes to be targeted. Therefore, there has been a problem that it is difficult to select a gene useful as a drug discovery target among those candidate genes.
Patent Document 1: WO 03/030725
Patent Document 2: WO 02/86443
Non-patent Document 1: Vogel C, Cobleigh MA, Eur J Cancer. 2001 Jan; 37 Suppl 1: 25-29
Non-patent Document 2: J. Baselga, Clinical trials of Herceptin, Eur. J. Cancer 37 Suppl 1(2001)S18-24.

### DISCLOSURE OF THE INVENTION

### Problems to be solved by the Invention

An object of the present invention is to provide a novel nucleotide sequence and an use of a cancer-related gene. Another object of the present invention is to provide a method of screening a drug using the cancer-related gene.

### Means for solving the Problems

As a result of intensive studies aiming at providing a novel nucleotide sequence useful as a drug discovery target, the inventors of the present invention have identified novel nucleotide sequences showing an increased expression in cancer cells or cancer tissues as compared with normal tissues. As a result of further investigation, the inventors of the present invention have found cancer-related genes which are useful as a target for drug discovery and show an increased expression in cancer cells or cancer tissues, thereby completing the present invention.

That is, the present invention is as described below.
(1) A preventive and/or therapeutic agent for lung cancer, comprising an antisense DNA against a DNA consisting of a nucleotide sequence represented by any one of SEQ ID NOS: 1 to 4 in the Sequence Listing.
(2) A preventive and/or therapeutic agent for lung cancer, comprising an antibody against a polypeptide encoded by a DNA consisting of a nucleotide sequence represented by any one of SEQ ID NOS: 1 to 4 in the Sequence Listing.
(3) The preventive and/or therapeutic agent for lung cancer according to (2), wherein the antibody is a monoclonal antibody.
(4) The preventive and/or therapeutic agent for lung cancer according to (2), wherein the antibody is a human monoclonal antibody.
(5) A DNA comprising a nucleotide sequence represented by SEQ ID NO: 5 in the Sequence Listing.
(6) A recombinant vector, comprising the DNA according to (5).
(7) A transformant, comprising the recombinant vector according to (6).
(8) A polypeptide encoded by the DNA according to (5).
(9) An antisense DNA against the DNA according to (5).
(10) A preventive and/or therapeutic agent for pancreatic cancer, comprising the antisense DNA according to (9).
(11) An antibody against the polypeptide encoded by the DNA according to (5).
(12) The antibody according to (11), wherein the antibody is a monoclonal antibody.
(13) The antibody according to (11), wherein the antibody is a human monoclonal antibody.
(14) A preventive and/or therapeutic agent for pancreatic cancer, comprising the antibody according to any one of (11) to (13).
(15) A method of screening a substance for inhibiting expression of the DNA according to (5).
(16) A method of screening a substance for inhibiting a function of a polypeptide encoded by the DNA according to (5).
(17) The method of screening according to (15) or (16), wherein the substance is a preventive and/or therapeutic agent for pancreatic cancer.

### Advantages of the Invention

According to the present invention, novel nucleotide sequences and use of cancer-related genes useful as a drug discovery target are provided. In addition, a method of screening a drug using the cancer-related genes is provided.

### DESCRIPTION OF THE PREFERRED EMBODIMENTS

Hereinafter, the present invention will be described in detail.
(1) Selection of nucleotide sequences to be used as a target
   Nucleotide sequences of SEQ ID NOS: 1 to 4 were selected by the following procedures using an expression profile database, BioExpress (GeneLogic Co., Ltd.) and an analysis software, GeneExpress™ Explorer version 1.4.

First, sets of samples were respectively prepared by selecting normal lung tissues, pulmonary adenocarcinoma tissues or pulmonary adenocarcinoma which has high expression of high-mobility group protein 4 (HMG4), which has been known to show enhanced expression in a pulmonary adenocarcinoma. A fold-change analysis (GeneExpress™ Explorer version 1.4) found 80 candidate probes whose average signal values were not less than 50 and enhanced in the pulmonary adenocarcinoma tissue three-times or more as compared with the normal lung tissue. Similarly, a fold-change analysis (GeneExpress™ Explorer version 1.4) found 307 candidate probes whose average signal values were not less than 50 and enhanced in the HMG4 high-expression lung cancer tissue three-times or more as compared with the normal lung tissue.

In addition, for further restricting normal lung tissues, an additional set of samples of normal lung tissues, excluding the cases in which primary disease of patients was associated with lung, were prepared. Then, 464 candidate probes whose average signal values were not less than 50 and enhanced in the above-mentioned pulmonary adenocarcinoma or HMG4 highly-expressing pulmonary adenocarcinoma three-times or more as compared with the set of samples of normal lung tissues were obtained.

Further, to confirm the expression level of those candidate probes in normal tissues except lung, the expression was analyzed in each of various normal tissues (e.g., breast, duodenum, esophagus, heart, kidney, liver, muscle, ovary, pancreas, skin, small intestine, large intestine, rectum, spleen, stomach, thymus, uterus cervix, endometrium, myometrium, prostate, lymph node, lymphocyte, and leucocyte). Among candidate probes, probes having higher expression level in at least one of those various normal tissues other than reproductive tissues as compared with that of pulmonary adenocarcinoma were excluded. Consequently, 4 probes from the above 80 probes, 145 probes from the 307 probes, and 23 probes from the 464 probes were selected.

Further, for those candidates, prediction on the secondary structure and transmembrane region were made by SOSUI (Bioiniformatics, 14(4), 378-379, (1998)) and TMHMM (Journal of Molecular Biology, 305(3): 567-580 (2001)). As a result, candidate genes which may be of membrane proteins including "similar to monocarboxylate transporter 4" (Probe Set ID: 238029_s_at; SEQ ID NOS: 1 and 2) and "hypothetical protein" (Probe Set ID: 227804_at; SEQ ID NO: 3) were obtained. In addition, "ESTs" (Probe Set ID: 241031_at; SEQ ID NO: 4), whose amino acid sequence was unknown, was also obtained. Further, amino acid sequences of SEQ ID NOS: 6, 7, and 8, which respectively correspond to SEQ ID NOS: 1, 2, and 3, were obtained.

For "similar to monocarboxylate transporter 4" (238029_s_at) of SEQ ID NOS: 1 and 2, there is a report that describes an increase of its expression in prostate cancer (WO 0160860, Millennium Predictive Medicine Co., Ltd.), but there has not been any report that describes an increase of its expression in lung cancer cells or tissues.

For "hypothetical protein" (227804_at) of SEQ ID NO: 3, there is neither report that describes an increase of its expression in cancer cells or tissues, nor report that describes an increase of its expression in lung cancer cells or tissues.

For "ESTs" (Probe SET ID: 241031_at) of SEQ ID NO: 4, there is neither report that describes an increase in its expression in cancer cells or tissues, nor report that describes an increase of its expression in lung cancer cells or tissues.

The nucleotide sequence of SEQ ID NO: 5 was selected by the following procedures.

Sample sets of normal pancreatic tissue excluding the cases in which primary diseases were associated with the pancreas, and sample sets of pancreatic ductal carcinoma were prepared. About 2,700 probes, which showed positive expression rate of 70% or more in a pancreatic ductal cancer tissue and 30% or less in a normal pancreatic tissue by a signature differential analysis, were obtained. In addition, for those probes, the expression was analyzed in each of various normal tissues except pancreas (e.g., bladder, breast, duodenum, esophagus, heart, kidney, liver, lung, muscle, ovary, prostate, salivary gland, skin, small intestine, large intestine, spleen, stomach, testis, thymus, thyroid, endometrium, lymph node, lymphocyte, and leucocyte). Among candidate probes, probes having higher expression level in at least one of those various normal tissues other than reproductive tissues as compared with that of pancreatic ductal carcinoma were excluded, thereby obtaining 42 probes. Of those, ESTs (229479_at) whose function was unknown were found.

The ESTs (229479_at) has been known to have increased expression in colon cancer (WO 0122920) and breast cancer (WO 0078960 and WO 0259271), but its increased expression in pancreatic cancer has not yet been reported.
For the EST (229479_at), the accession number in UniGene database (Nucleic Acids Res., 31(1), 28-33 (2003)) of a gene corresponding to Affymetrix Probe Set ID 229479_at was searched by means of annotation in BioExpress database. As a result, Accession No. Hs. 152812 was found to be the accession number of an EST cluster corresponding to the EST (229479_at). The UniGene database search found that the nucleotide sequence of the ESTs belonging to Hs. 152812 cluster registered in public database includes each of the nucleotide sequence of GenBank Accession Nos. AI538632, AA513096, AI739132, AI123717, AI740516, AI467852, AI741376, AW044211, AA631257, AA464510, BQ002341, AW972467, AW205510, AW028889, AW198033, AW068935, AI754551, AI752240, and CB044990. Among these nucleotide sequence of the EST cluster, some nucleotide sequences have an additional poly-A sequence. In addition, the chromosomal position of the ESTs was found to be located at 2q35 on chromosome 2.

As a result of homology search for the nucleotide sequence represented by GenBank Accession No. AI740516 using BLAST (Basic local alignment search tool; Altschul, S. F., et al., J. Mol. Biol., 215, 403-410 (1990)), there was a hit against the nucleotide sequence represented by Accession No. AAH34731 with 100% identity over 591 nucleotides and against the nucleotide sequence represented by Accession No. ABT10488 with 98% identity over 572 nucleotides, in the nucleotide sequence database (nageneseq) in a Patent sequence database (GeneSeq).

Further, as a result of homology search for the nucleotide sequence represented by Accession No. AAH34731 using BLAST, there was a hit with the nucleotide sequence represented by Accession No. AAF44952 with 100% identity over 225 nucleotides, and against the nucleotide sequence represented by Accession No. ABL82489 with 99% identity over 353 nucleotides, in the nucleotide sequence database (nageneseq) in the Patent sequence database (GeneSeq).

Further, as a result of homology search for the nucleotide sequence represented by Accession No. AAF44952 using BLAST, there was a hit with the nucleotide sequence represented by Accession No. CB044991 with 100% identity over 80 nucleotides, and against the nucleotide sequence represented by Accession No. CB045367 with 100% identity over 318 nucleotides, in the nucleotide sequence database (nageneseq) in the Patent sequence database (GeneSeq).

Each of the nucleotide sequences represented by GenBank Accession Nos. AI538632, AA513096, AI739132, AI123717, AI740516, AI467852, AI741376, AW044211, AA631257, AA464510, BQ002341, AW972467, AW205510, AW028889, AW 198033, AW068935, AI754551, AI752240, CB044990, CB045367, and CB044991, and the nucleotide sequences represented by Accession Nos. AAH34731, ABT10488, and AAF44952 from the nucleotide sequence database (nageneseq) in the Patent sequence database (GeneSeq) was mapped on the sequence of chromosome 2 from human genome sequence NCBI build 31 by means of a software sim4 (Genome Res., 8, 967-974 (1998)) for mapping a cDNA nucleotide sequence to a genome nucleotide sequence. As a result, the nucleotide sequences of all ESTs were mapped on contig nucleotide sequence of GenBank Accession No. AC093850 of chromosome 2.
Nucleotide sequences represented by GenBank Accession Nos. CB044991 and CB044990 corresponded to the nucleotide sequence from 5'-end and 3'-end of the nucleotide sequence represented by Accession No. 3218160 of clones from the I.M.A.G.E. consortium, respectively. The nucleotide sequence represented by Accession No. CB044991 having one splice site was mapped with intervening an intron, while the nucleotide sequence represented by Accession No. CB044990 was mapped without having any splice site. These two nucleotide sequences were mapped without overlapping each other, and were mapped over 2,900 nucleotides with an interval of 1,027 nucleotides therebetween on the genome. The nucleotide sequence represented by Accession No. CB044991 was mapped such that it had a region at the 3'-end which overlapped with the nucleotide sequence represented by Accession No. AAF44952. The nucleotide sequence represented by Accession No. AAF44952 was mapped such that it had a region at the 3'-end which overlapped with the nucleotide sequence represented by Accession No. AAH34731. The nucleotide sequence represented by Accession No. AAH34731 was mapped such that it had an overlapped region with each of the nucleotide sequences represented by Accession Nos. ABT10488, ABL82489, CB045367, AI538632, AA513096, AI739132, AI123717, AI740516, AI467852, AI741376, AW044211, AA631257, AA464510, BQ002341, AW972467, AW205510, AW028889, AW198033, AW068935, AI754551, AI752240, and CB044990. Of those, CB044991 was found to be a nucleotide sequence on the farthest 5'-end part.

When homology search for the nucleotide sequence represented by Accession No. CB044991 was performed by using BLAST, no nucleotide sequence overlapping with the sequence represented by Accession No. CB044991 on the 5'-end part thereof was detected. Therefore, the nucleotide sequence represented by Accession No. CB044991 has been considered as a nucleotide sequence on the farthest 5'-end part of the sequence of the gene registered in a public database and the patent sequence database until now.

The nucleotide sequence of the probe of Affymetrix Probe Set ID 229479_at was searched using NetAffyx database (Nucleic Acids Res., 31(1), 82-86 (2003)). As a result of mapping the nucleotide sequence of the probe on the contig sequence of AC093850 using sim4, the nucleotide sequence of the probe was found to be mapped on the region of about 500 nucleotides on the 3'-end part in the region on which the ESTs were mapped.

The nucleotide numbers from 20,000 to 23,100 of the genome sequence (Accession No. AC093850 of GenBank database); nucleotide sequences represented by GenBank Accession Nos. AI538632, AA513096, AI739132, AI123717, AI740516, AI467852, AI741376, AW044211, AA631257, AA464510, BQ002341, AW972467, AW205510, AW028889, AW198033, AW068935, AI754551, AI752240, CB044990, CB045367, and CB044991; and nucleotide sequences represented by Accession Nos. AAH34731, ABT10488, and AAF44952 in the nucleotide sequence database (nageneseq) of the Patent sequence database (GeneSeq) were subjected to clustering by using the SeqMan program available from DNASTAR Inc., and then a nucleotide sequence of the longest virtual EST was obtained as a novel nucleotide sequence of SEQ ID NO: 5 on the basis of the nucleotide sequence of Accession No. AC093850 of the GenBank database.

There has been no report on an increase in expression of the novel nucleotide sequence of SEQ ID NO: 5 in pancreas cancer cells or tissues.

The nucleotide sequence of SEQ ID NO: 5 consists of 2,184 nucleotides in total length. As a result of consideration together with the result of mapping by the above-mentioned sim4, a splice site was found between nucleotide 220 and nucleotide 221, so it was assumed that an intron's nucleotide sequence is present between these nucleotides in an immature mRNA.

A genewise program (http://www.ebi.ac.uk/Wise2/) was employed for searching the nucleotide sequence of SEQ ID NO: 5 in the protein profile database Pfam (Nucleic Acids Res. 30(1) 276-280 (2002)). However, no profile for a functional domain of protein was detected.

As a result of search of an open reading frame of the above sequence using the NCBI ORF Finder (http://www.ncbi.nlm.nih.gov/gorf/gorf.html), the longest open reading frame had 177 nucleotides. The possible reasons why an open reading frame having 300 or more nucleotides could not be detected are as follows: the nucleotide sequences of ESTs registered in the public database and patent sequence database until now are 3'-end noncoding regions in mRNA; a sequence in which mutations, frame shift, or the like have occurred in an actual sequence is registered; the gene functions as a gene encoding a peptide of not more than 100 amino acid residues or as a RNA; and so on.
(2) Nucleotide Sequence and DNA
   In the present invention, a DNA consisting of a nucleotide sequence represented by any of SEQ ID NOS: 1 to 5 in the Sequence Listing encompasses a DNA consisting of a nucleotide sequence which hybridizes with a complementary strand of the DNA represented by the aforementioned nucleotide sequence under stringent conditions. In the present invention, a nucleotide sequence represented by any of SEQ ID NOS: 1 to 5 in the Sequence Listing or a nucleotide sequence which hybridizes with a complementary strand of a polynucleotide represented by the aforementioned nucleotide sequence under stringent conditions may be abbreviated as "nucleotide sequence or the like".

In the present invention, a nucleotide sequence which hybridizes under stringent conditions encompasses a nucleotide sequence having not less than 80%, preferably not less than 90%, or more preferably not less than 95% homology with a complementary strand of a nucleotide sequence represented by any of SEQ ID NOS: 1 to 5. Hybridization can be carried out according to a known method or a method pursuant thereto, such as one described in Molecular Cloning 2nd (J. Sambrook et al., Cold Spring Harbor Lab. Press, 1989). Examples of a stringent condition includes conditions in which a sodium concentration is about 19 to 40 mM, preferably 19 to 20 mM, and a temperature is about 50 to 70°C, preferably about 60 to 65°C. In particular, the most preferable condition includes a sodium concentration of about 19 mM and a temperature of 65°C.

Further, the nucleotide sequence of the present invention encompasses a sequence which has not less than 50% identity with a nucleotide sequence represented by any of SEQ ID NOS: 1 to 5. It preferably encompasses a nucleotide sequence having an identity of not less than 60%, more preferably not less than 70%, still more preferably not less than 80%, further more preferably not less than 90%, and much more preferably not less than 95% with the nucleotide sequence. In addition, the nucleotide sequence of the present invention encompasses a nucleotide sequence represented by any of SEQ ID NOS: 1 to 5 including insertion, deletion, or substitution of nucleotides. Here, the number of nucleotides inserted, deleted, or substituted may be one nucleotide or two or more nucleotides, for example, 1 to 30 nucleotides, preferably 1 to 10 nucleotides, and more preferably 1 to 5 nucleotides. In this case, the nucleotide sequence has the same action or function as each of the SEQ ID NOS: 1 to 5, even when the nucleotide sequence has insertion, deletion, or substitution of nucleotides. (3) Recombinant vector and transformant
A recombinant vector to be used in the present invention may be a vector (e.g., pBR322, pUC119, or a derivative thereof) which can be expressed in a prokaryotic cell such as *Escherichia coli.* Preferably, the vector may be one which can be expressed in a eukaryotic cell. Examples of vectors which can be expressed in cells derived from mammals include plasmid vectors such as pcDNA3.1 (Invitrogen Co., Ltd.) and virus vectors such as pDON-Al DNA (Takara Bio Inc.).

The recombinant vector of the present invention is any one of the above-mentioned vectors into which a whole or partial sequence of the DNA having the nucleotide sequence of the present invention is recombined by conventional procedures.

The transformant of the present invention means a transformant comprising the recombinant vector of the present invention. The transformant may be a prokaryotic cell such as *Escherichia coli,* but preferably a eukaryotic cell, and more preferably a mammal-derived cell. An example of mammal-derived cell includes Chinese hamster ovary cell (i.e., CHO cell).
(4) Polypeptide
   In the present invention, polypeptides refer to those generated by transcription and translation of a gene consisting of a nucleotide sequence represented by SEQ ID NO: 5 and so on. The polypeptide most preferably includes one generated by transcription and translation of a gene consisting of a nucleotide sequence represented by SEQ ID NO: 5, but also includes a polypeptide having the amino acid sequence with deletion, addition, insertion or substitution of one or two amino acids, and a polypeptide having the amino acid sequence with a combination thereof. When amino acids are inserted, deleted, or substituted as described above, the position of insertion, deletion, or substitution is not limited.
(5) Antisense DNA
   In the present invention, antisense DNA means a DNA having a nucleotide sequence which can inhibit the function of a polypeptide encoded by a DNA consisting of a nucleotide sequence represented by any of SEQ ID NOS: 1 to 5 in the Sequence Listing. The antisense DNA of the present invention complementally binds to the DNA of the present invention to inhibit the expression of the DNA of the present invention. Therefore, the antisense DNA of the present invention can be used as a preventive and/or therapeutic agent for cancer.
(6) Antibody
   In the present invention, antibody includes a polyclonal antibody from various animals and a monoclonal antibody from various animals.

Examples of monoclonal antibody include a mouse monoclonal antibody, a human-mouse chimera antibody, a humanized monoclonal antibody, and a human monoclonal antibody. Of those, a human monoclonal antibody is preferable. More preferable is a cancer-reactive human monoclonal antibody. Further, examples of the monoclonal antibody include monoclonal antibodies, fragments of monoclonal antibody, F(ab')₂ antibodies, Fab antibodies, short-chain antibodies (scFv), diabodies, tribodies, and minibodies. When the monoclonal antibody contains a constant region, an amino acid sequence of constant region in heavy and light chains are preferably those described in Nucleic Acids Research vol.14, p1779, 1986, The Journal of Biological Chemistry vol.257, p1516, 1982 and Cell vol.22, p197, 1980. In addition, examples of an antibody include whole antibody (e.g., whole-length antibody and entire antibody), antibody fragments (e.g., fragment of antibody such as Fab, F(ab')₂, scFv (i.e., single-chain antibody)), or derivatives of the antibodies. More preferable is F(ab')₂ antibody.
(7) Screening method
   The present invention provides a method of screening a preventive and/or therapeutic agent for lung cancer, which inhibits the expression of a DNA consisting of the nucleotide sequence represented by any of SEQ ID NOS: 1 to 4 in the Sequence Listing. In addition, the present invention provides a method of screening a substance for inhibiting the expression of a DNA consisting of the nucleotide sequence represented by SEQ ID NO: 5 in the Sequence Listing. Here, examples of the substance include preventive and/or therapeutic agent for cancer, and preferably include preventive and/or therapeutic agent for pancreatic cancer. As a screening method, screening on the basis of binding of the nucleotide sequence or the like of the present invention with a test substance can be carried out. The binding of the nucleotide sequence or the like of the present invention with the test substance can be easily detected by fixing one of them on a solid phase and labeling the other. Thus, for example, a procedure using a combinatorial library synthesized on a support is suitable for the screening of the present invention. In this case, a fluorescence-labeled nucleotide sequence or the like of the present invention is allowed to react with the test substance, thereby easily detecting the binding between them.

The present invention provides a method of screening a preventive and/or therapeutic agent for lung cancer, which inhibits the function of a polypeptide encoded by a DNA consisting of a nucleotide sequence represented by any of SEQ ID NOS: I to 4 in the Sequence Listing. In addition, the present invention provides a method of screening a substance for inhibiting the function of a polypeptide encoded by a DNA consisting of the nucleotide sequence represented by SEQ ID NO: 5 in the Sequence Listing. As a screening method, screening on the basis of binding of the polypeptide encoded by the DNA of the present invention with a test substance can be carried out. The binding of the polypeptide encoded by the DNA of the present invention with the test substance can be easily detected by fixing one of them on a solid phase and labeling the other. Thus, for example, a procedure using a combinatorial library synthesized on a support is suitable for the screening of the present invention. In this case, a polypeptide encoded by the DNA of the present invention is fluorescence-labeled and then reacted with a test substance, so the binding between them can be easily detected.

Examples of the preventive and/or therapeutic agent for lung cancer and the substance, which can be obtained by the screening method of the present invention, include low-molecular-weight compounds and high-molecular-weight compounds.

Examples of the high-molecular-weight compounds include polynucleotides, polypeptides, and ligands. Of those, the ligands are preferable.

In the present invention, the ligands include those that bind to target cells or target molecules. Here, examples of the target cells include cancer cells, vascular endothelial cells in cancer tissues, and interstitial cells in cancer tissues. In addition, examples of the target molecules may be any of cytoplasmic molecules, intranuclear molecules, and cell-surface molecules in the target cells. Of those, cell-surface molecules are preferable. Another type of the target molecules includes molecules that are to be released from cells, such as molecules secreted from cancer cells or interstitial cells in cancer tissues and structural molecules thereof. Specific examples thereof include tumor markers and intercellular structural molecules. More specifically, ligands include those that bind to the target cells or target molecules as described above, and examples thereof include proteins including antibodies and growth or proliferative factors such as fibroblast growth factor (FGF) and epidermal growth factor (EGF). Of those, antibodies are preferable.

Further, a ligand is capable of specifically recognizing the polypeptide of the present invention, so it can be used for quantitative determination of the polypeptide of the present invention in a test solution, such as quantitative determination by sandwich immunoassay, in addition to a case where it is used as a preventive and/or therapeutic agent. In addition, it can also be used for preparing an antibody column to be employed for purification of the polypeptide of the present invention, detection of the polypeptide of the present invention in each fraction during purification, and analysis of the behavior of the polypeptide of the present invention in a test cell.
(8) Drug formulation and dosage
   When the antisense DNA of the present invention is used as a preventive and/or therapeutic agent for cancer, the antisense DNA can be formulated and administered according to known methods. For example, when the aforementioned antisense DNA is used, the antisense DNA which has been inserted into a suitable vector such as a retroviral vector, an adenoviral vector, or an adenovirus-associated virus vector in advance, or the antisense DNA alone can be orally administered or parenterally administered to a human or a mammal (e.g., rat or rabbit) according to known methods. The antisense DNA of the present invention may be administered to a human at a dosage of 0.01 to 0.1 mg/ml.

When the antibody of the present invention is used as a preventive and/or therapeutic agent for cancer, the antibody can be formulated and administered according to known methods. For example, the antibody can be orally or parenterally administered to a human or a mammal directly in a liquid form or a pharmaceutical composition of a suitable dosage form. The antibody of the present invention may be administered to a human at a dosage of 0.01 to 1.0 mg/kg.

When the low-molecular-weight compound of the present invention is used as a preventive and/or therapeutic agent for cancer, the low-molecular-weight compound can be formulated and administered according to known methods. For example, the low-molecular-weight compound can be orally or parenterally administered to a human or a mammal as a pharmaceutical composition in a suitable dosage form such as a liquid, tablet, or capsule formulation. The low-molecular-weight compound of the present invention may be administered once or more times per day to a human at a dosage of 0.5 to 300 mg/kg body weight.

The dosage varies depending on age, body weight, general physical conditions, sexuality, meals, time of administration, a method of administration, a rate of excretion, a combination of drugs, and the degree of conditions of a patient being treated at the moment, and the dosage is determined in consideration of any of these or other factors.

Examples of pharmaceutical compositions suitable for oral administration include tablet, capsule, powder, liquid, and elixir formulations. Examples of pharmaceutical compositions suitable for parenteral administration include pharmaceutical compositions in a sterilized liquid form such as liquid formulation or suspension.

Another example of the preventive and/or therapeutic agent for cancer includes a kit for gene therapy. In the present invention, the gene therapy kit may be a kit used for killing or damaging lung cancer cells or pancreatic cancer cells, and a therapeutic kit used for treating cancer. The gene therapy kit contains a suitable container, a pharmaceutical formulation of a recombinant vector capable of expressing an antisense DNA such as a nucleotide sequence of any of SEQ ID NOS: 1 to 5 in an animal cell, and a pharmaceutical formulation of a DNA damaging agent. Here, the recombinant vector and the DNA damaging agent may be provided in a single container or may be provided in different or separated containers. For instance, the recombinant vector may be a recombinant adenoviral vector in which the antisense DNA is present in an adenovirus particle, while the DNA damaging agent may be cisplatin. The constituents of the kit can be provided preferably in a form of liquid solution or dry powder. When the constituents are provided in a form of liquid solution, the liquid solution is aqueous solution and preferably sterilized aqueous solution. When a reagent or constituent is provided as a dry powder, the powder can be reconstituted by addition of an appropriate solvent. The solvent may be provided by means of other containers.

### EXAMPLES

Hereinafter, the present invention will be described in detail with reference to examples. However, the present invention is not limited to the examples described below.
(1) Cell lines used
   For gene expression analysis, cell lines to be used include: PC-3 (Immuno-Biological Laboratories Co., Ltd.), NCI-H522 (ATCC No. CRL-5810), and A549 (ATCC No. CRL-185), which are derived from lung cancer; HPAC (ATCC No. CRL-2119), PANC-1 (ATCC No. CRL-1469), and SUIT-2 (available from National Kyushu Cancer Center), which are derived from pancreatic cancer; MCF7 (ATCC No. HTB-22), which is derived from breast cancer; HCT-15 (ATCC No. CCL-225) and HCT-116 (ATCC No. CCL-247), which are derived from colon cancer; U937 (ATCC No. CRL-1593.2), which is derived from histocytic lymphoma; and VA-13 (ATCC No. CCL-75.1), which is derived from a fibroblast.
(2) RNA extraction from cancer cell lines and reverse transcription reaction
   Extraction of total RNA from various cell lines is carried out using TRIzol Reagent (Invitrogen Co., Ltd.) in accordance with an attached manual. The total RNA is treated with RNase-free DNase I (Roche Diagnostics Co., Ltd.) for 15 minutes at 37°C. Subsequently, ethanol precipitation is carried out using 1/10 volume of 3 M NaOAc and 2.5 volume of ethanol, thereby concentrating and recovering the total RNA. A reverse transcription reaction of the total RNA (200 ng) is carried out using oligo-dT or random hexamer as a primer, and using a superscript II reverse transcriptase from Invitrogen Co., Ltd. in accordance with an attached manual.
(3) PCR method
   PCR is carried out using as a template 10 ng of a cDNA derived from each of various cell lines, which is synthesized by a reverse transcription reaction.

PCR is carried out under the conditions in which 2-minute treatment at 96°C is carried out first, and then a cycle of 96°C, 30 seconds, 60°C, 30 seconds, and 72°C, 1 minute is repeated 35 times, followed by lowering to 4°C.
Sequences of primers used in the PCR analysis are SEQ ID NOS: 9 and 10 for SEQ ID NOS: 1 and 2, and SEQ ID NOS: 11 and 12 for SEQ ID NO: 5, respectively.
(4) Northern Analysis
   For Northern analysis, total RNA or poly-A RNA extracted from the various cell lines is used. The purification of poly-A RNA from total RNA is carried out using Oligotex-dT30 <Super> mRNA purification kit available from Takara Bio Inc.

A series of operations for Northern analysis (i.e., agarose electrophoresis, capillary transfer to a nylon membrane, preparation of an RNA probe or a DNA probe which are labeled with digoxigenin (DIG), hybridization and washing, and detection by chemiluminescence) is carried out in accordance with a DIG application manual prepared by Roche Diagnostics Co., Ltd. Specifically, 1 µg of total RNA or 100 ng of poly-A RNA, which are derived from each of the various cell lines, is mixed with MOPS buffer, deionized formamide, formaldehyde, loading buffer, and ethidium bromide, and then denatured by treating for 10 minutes at 65°C. Subsequently, the mixture is added to a denatured gel containing 1% agarose and then subjected to electrophoresis (100 V, about 1 hour) together with an RNA molecular weight marker. The gel is washed twice in 20 × SSC buffer for 15 minutes per each time, and then subjected to capillary transfer to a positive-charged nylon membrane (Roche Diagnostics Co., Ltd.) (for 16 hours). The membrane is subjected to UV-crosslink (120 mJ) and then air-dried. Subsequently, the membrane is subjected to pre-hybridization in 5 ml of hybridization buffer (DIG Easy Hyb, Roche Diagnostics Co., Ltd.) at 68°C for 1 hour. The hybridization is carried out using 20 to 100 ng/ml of an RNA probe at 68°C overnight. Further, RNA probes used for detecting mRNAs of SEQ ID NO: 1 (MCT4-like protein) and SEQ ID NO: 5 (Pancreatic cancer Est, 229479_at) are an antisense RNA (743 bp) against the nucleotides from 2,715 to 3,457 counted from the 5' end of the nucleotide sequence of SEQ ID NO: 1, and an antisense RNA (725 bp) against the nucleotides from 1,312 to 2,036 counted from the 5' end of the nucleotide sequence of SEQ ID NO: 5, respectively.

After the hybridization, the membrane is washed in a high-stringency buffer (2 × SSC, 0.1 % SDS) for 5 minutes at room temperature twice. Then, the membrane is washed in low-stringency buffer (0.1 × SSC, 0.1% SDS) at 68°C for 15 minutes twice. Subsequently, after the hybridization, blocking and washing of the membrane are carried out using DIG Wash and Block Buffer Set and anti-DIG- alkaline phosphatase (Roche Diagnostics Co., Ltd.). In addition, CDP-Star (Roche Diagnostics Co., Ltd.) is used as a substrate for detecting chemiluminescence and LAS2000 (FUJIFILM Co., Ltd.) is used as a detector. (5) Preparation of antiserum against a gene product
From an amino acid sequence (hereinafter, also referred to as "MCT14") encoded by a DNA consisting of the nucleotide sequence represented by SEQ ID NO: 1, a peptide consisting of 18 amino acids from the C-terminal (SEQ ID NO: 14 in the Sequence Listing) was bound to Keyhole Limpet Haemocyanin (KLH) to immunize a rabbit, thereby obtaining antiserum.

An anti-MCT14 peptide antibody was obtained by purifying the antiserum by using an affinity column where the above-mentioned peptide was immobilized on CNBr Sepharose (GE Healthcare Bio-Sciences K.K.).
(6) Detection and Western blotting of a gene product using antiserum
   (6-1) Preparation of MCT14 gene expression vector
      PCR primers were designed from the sequence information of SEQ ID NO: 1 in the Sequence Listing, and PCR was then carried out according to the method described above using, as a template, cDNA of a human cancer cell line, NCI-H522 (lung cancer cell line), HCT-15 (colon cancer cell line), or MCF-7 (breast cancer cell line). The sequences of the resulting PCR products were analyzed by cloning them in PCR-BluntII-TOPO (Invitrogen Co., Ltd.), thereby confirming that each of the clones corresponded to SEQ ID NO: 1 in the Sequence Listing. Further, from those vectors, an expression vector in which c-myc was added to the MCT14 gene product was prepared.
   (6-2) Preparation of transient expression cell and confirmation of reactivity of anti-MCT14 peptide antibody
      The expression vector was transfected to COS-7 cells using Lipofectamin 2000 (Invitrogen Co., Ltd.) and the cells were then collected 24 hours later. After that, the cells were solubilized in a buffer containing 20 mM Tris-HCl (pH 7.4), 150 mM NaCl, 1mM EDTA, 1% Brij97, 2.5 mM iodoacetic acid amide, and Complete (Roche Diagnostics Co., Ltd.) as a protease inhibitor, and a supernatant was obtained. The solubilized supernatant was subjected to precipitation with acetone and the precipitated protein was then dissolved in 8 M urea and SDS-PAGE sample buffer, followed by SDS-PAGE/Western blotting operation using the sample. A protein concentration in each solubilized supernatant was determined by BCA protein assay kit (PIERCE Co., Ltd.) in advance, thereby adjusting the amount of the protein applied on the SDS-PAGE to be constant.

After blotting, blocking was carried out using PBS containing 0.1% gelatin and 0.05% Tween20.

First, for confirming the expression of a gene product of MCT14, the membrane was reacted at room temperature for 1 hour in a solution containing 1,000-fold diluted anti-c-myc monoclonal antibody (Invitrogen Co., Ltd.) and then reacted at room temperature for 1 hour in a solution containing 1,000-fold diluted HRP-labeled anti-mouse IgG (DAKO Co., Ltd.). Further, for confirming the reaction of an anti-MCT14 peptide antibody with the MCT14 gene product, it was reacted with 3 µg/ml of anti-MCT14 peptide antibody at room temperature for 1 hour, and then reacted with 1,000-fold diluted HRP-labeled anti-rabbit IgG (Capell Co., Ltd.) at room temperature for 1 hour.

Bands were detected using ECL (GE Healthcare Bio-Sciences K.K.).

A competitive experiment with a peptide was carried out using a primary antibody solution prepared by adding a peptide of SEQ ID NO: 14 in the Sequence Listing into a solution containing 3 µg/ml of anti-MCT14 peptide antibody so that the final concentration of the peptide became 1 µg/ml or 5 µg/ml and then incubating the solution at 4°C overnight, and the detection was performed using HRP-labeled anti-rabbit IgG.

On lane 1, solubilized supernatant of the cells in which the MCT14 gene had been introduced was applied. After that, the presence or absence of a band was detected using a normal rabbit IgG. On lane 2, solubilized supernatant of cells without gene introduction (Mock cells) was applied. After that, the presence or absence of a band was detected using a normal rabbit IgG. On lane 3, solubilized supernatant of the cells in which the MCT14 gene had been introduced was applied. After that, the presence or absence of a band was detected using an anti-MCT14 peptide antibody. On lane 4, solubilized supernatant of the Mock cells was applied. After that, the presence or absence of a band was detected using an anti-MCT14 peptide antibody. On lane 5, solubilized supernatant of the cells in which the MCT14 gene had been introduced was applied. After that, the presence or absence of a band was detected using a solution which had been prepared by adding a peptide of SEQ ID NO: 14 in the Sequence Listing at a final concentration of 1 µg/ml into the anti-MCT14 peptide antibody and incubating them. On lane 6, solubilized supernatant of the Mock cells was applied. After that, the presence or absence of a band was detected using a solution which had been prepared by adding a peptide of SEQ ID NO: 14 in the Sequence Listing at a final concentration of 1 µg/ml into the anti-MCT14 peptide antibody and incubating them. On lane 7, solubilized supernatant of cells in which the MCT14 gene had been introduced was applied. After that, the presence or absence of a band was detected using a solution which had been prepared by adding a peptide of SEQ ID NO: 14 in the Sequence Listing at a final concentration of 5 µg/ml into the anti-MCT14 peptide antibody and incubating them. On lane 8, solubilized supernatant of the Mock cells was applied. After that, the presence or absence of a band was detected using a solution which had been prepared by adding a peptide of SEQ ID NO: 14 in the Sequence Listing at a final concentration of 5 µg/ml into the anti-MCT14 peptide antibody and incubating them. On lane 9, solubilized supernatant of the cells in which the MCT14 gene had been introduced was applied. After that, the presence or absence of a band was detected using an anti-c-myc antibody.

Consequently, a band that reacted with the anti-c-myc antibody was detected at the position of about 50 kDa (lane 9), so the expression of MCT14 in COS7 cells was confirmed. In addition, it was confirmed that the anti-MCT14 peptide antibody showed reactivity against the MCT14 gene product (lane 3). In addition, this reaction was competed with the peptide (lanes 5 and 7), so the reaction was confirmed to be specific to the MCT14 gene product.
(6-3) Expression of MCT14 protein in various cancer cell lines
   A supernatant was prepared by solubilizing each of human lung cancer cell line BT474 (BioExpress, signal level: 1,243), lung cancer cell line NCI-H460 (ditto.: 1,015), prostate cancer cell line LNCAP (ditto.: 535), and lung cancer cell line A549 (ditto. 48) in a buffer containing 20 mM Tris-HCl (pH 7.4), 150 mM NaCl, 1 mM EDTA, 1% Brij97, 2.5 mM iodoacetic acid amide, and Complete (Roche Diagnostics Co., Ltd.) as a protease inhibitor. Solubilized supernatant was subjected to precipitation with acetone and the precipitated protein was then dissolved in 8 M urea and SDS-PAGE sample buffer, followed by SDS-PAGE/Western blotting operation using the sample. A protein concentration in each solubilized supernatant was determined by BCA protein assay kit (PIERCE Co., Ltd.), thereby adjusting the amount of the protein applied on the SDS-PAGE to be constant. A membrane after blotting was blocked using PBS containing 0.1 % gelatin and 0.05% Tween20, and then reacted in 3 µg/ml of an anti-MCT14 peptide antibody at room temperature for 1 hour, followed by reacting in 1,000-fold diluted solution of HRP-labeled anti-rabbit IgG at room temperature for 1 hour. Bands were detected using ECL.

On lane 1, solubilized supernatant of COS7 cells without the introduction of the MCT14 gene was applied. On lane 2, solubilized supernatant of COS7 cells in which the MCT14 gene had been introduced was applied. On lane 3, solubilized supernatant of BT474 cells was applied. On lane 4, solubilized supernatant of NCI-H460 cells was applied. On lane 5, solubilized supernatant of LNCAP cells was applied. On lane 6, solubilized supernatant of A549 cells was applied.

Consequently, a band was detected at the same position as that of the band detected by transient expression in COS7 cells, so the DNA consisting of the nucleotide sequence of the present invention was revealed to be expressed as a peptide in cancer cells. Further, the higher the signal value in BioExpress, the thicker the detected band was. Therefore, it was revealed that the signal value in BioExpress correlated with the concentration of the band, and that the DNA consisting of the nucleotide sequence of the present invention can be actually used as a target for drug development.
(7) Detection and immunohistological staining of a gene product using an antiserum
   For inhibiting endogenic peroxidase activity in a frozen or paraffin-embedded section of cancer tissue or normal tissue, sections are processed by 3.0% H₂O₂/MeOH method (15 ml of 30% hydrogen peroxide + 135 ml of methanol) at room temperature for 25 minutes. After being washed 3 times with 0.05 M TBS containing 0.1% Tween20 for 5 minutes per each time, the sections are incubated in 5% normal goat antiserum at room temperature for 40 minutes for blocking. The sections are reacted with an antiserum against each of the gene products at room temperature for 90 minutes as a primary antibody, followed by washing three times with 0.05 M TBS containing 0.1 % Tween20 for 5 minutes per each time. Subsequently, as a secondary antibody reaction, it was reacted with peroxidase-labeled anti-rabbit IgG at room temperature for 30 minutes, and then washed three times with 0.05 M TBS containing 0.1% Tween20 on ice for 5 minutes per each time. Further, it was washed three times with 0.05 M TBS on ice for 5 minutes per each time. After color development with DAB, the product is washed with running water, and then subjected to dehydration, penetration, and embedding, followed by microscopic examination.

### INDUSTRIAL APPLICABILITY

According to the present invention, novel nucleotide sequences and use of cancer-related genes useful as a target for drug development are provided.

Further, the present application has been filed while claiming the priority of Japanese Patent Application No. 2004-102681.

## Claims

1. A preventive and/or therapeutic agent for lung cancer, comprising an antisense DNA against a DNA consisting of a nucleotide sequence represented by any one of SEQ ID NOS: 1 to 4 in the Sequence Listing.

2. A preventive and/or therapeutic agent for lung cancer, comprising an antibody against a polypeptide encoded by a DNA consisting of a nucleotide sequence represented by any one of SEQ ID NOS: 1 to 4 in the Sequence Listing.

3. The preventive and/or therapeutic agent for lung cancer according to claim 2, wherein the antibody is a monoclonal antibody.

4. The preventive and/or therapeutic agent for lung cancer according to claim 2, wherein the antibody is a human monoclonal antibody.

5. A DNA comprising a nucleotide sequence represented by SEQ ID NO: 5 in the Sequence Listing.

6. A recombinant vector, comprising the DNA according to claim 5.

7. A transformant, comprising the recombinant vector according to claim 6.

8. A polypeptide encoded by the DNA according to claim 5.

9. An antisense DNA against the DNA according to claim 5.

10. A preventive and/or therapeutic agent for pancreatic cancer, comprising the antisense DNA according to claim 9.

11. An antibody against the polypeptide encoded by the DNA according to claim 5.

12. The antibody according to claim 11, wherein the antibody is a monoclonal antibody.

13. The antibody according to claim 11, wherein the antibody is a human monoclonal antibody.

14. A preventive and/or therapeutic agent for pancreatic cancer, comprising the antibody according to any one of claims 11 to 13.

15. A method of screening a substance for inhibiting expression of the DNA according to claim 5.

16. A method of screening a substance for inhibiting a function of a polypeptide encoded by the DNA according to claim 5.

17. The method of screening according to claim 15 or 16, wherein the substance is a preventive and/or therapeutic agent for pancreatic cancer.
